# EUROPEAN PATENT APPLICATION

(11) **EP 0 687 688 A1**
(43) Date of publication of application: **20.12.1995**
(21) Application number: 95903822.5
(22) Date of filing: 17.12.1994
(51) Int. Cl.: C07K 16/12, C07K 5/10, C07K 14/315, C07K 14/33, A61K 39/395, G01N 33/569

(54) **ANTIBODIES AGAINST PNEUMOLYSINE AND THEIR APPLICATIONS**

(30) Priority: 17.12.1993 ES 9302615; 14.02.1994 ES 9400265; 13.08.1994 GB 9416393
(71) Applicant: UNIVERSIDAD DE OVIEDO, E-33003 Oviedo (ES); UNIVERSITY OF LEICESTER, Leicester, LE1 7RH (GB)
(72) Inventor: FLEITES, Ana, E-33001 Oviedo (ES); FERNANDEZ, Jesús M., E-28007 Madrid (ES); HARDISSON, Carlos, E-33002 Oviedo (ES); MENDEZ, Francisco J., E-33011 Oviedo (ES); DE LOS TOYOS, Juan R., E-33012 Oviedo (ES); VAZQUEZ, Fernando, E-33005 Oviedo (ES); MITCHELL, Timmothy, Swadlincote DE12 7AA (GB); ANDREW, Peter, Leicester LE2 3WF (GB); MORGAN, Peter J., Nottingham NG12 4EP (GB)
(74) Representative: Ibanez, José Francisco
(86) International application number: ES9400135
(87) International publication number: WO9516711

(57) **Abstract**

An object of the invention is the generation of hybridomas and the production and characterization of the corresponding monoclonal antibodies specific to pneumolysine, which could be used for various purposes, including diagnosis, prophylaxis and therapy. Amino acids sequences have also been identified, said sequences being required for cell binding and cytolysis. An other object of the invention is the detection of a cytolysine (pneumolysine) particularly in biological samples of patients, in order to diagnose the pneumococcyc infection, by means of enzymo-immunoassays of specific monoclonal antibodies. The method of the invention is characterized by an absolute specificity, is fast and very responsive.

## Description

The invention relates to the generation of hybridomas and to the production and characterization of the corresponding specific monoclonal antibodies (MAbs) for pneumolysin, which could have different applications, including diagnostic, prophylactic and therapeutic. Also the invention concerns amino acid sequences which comprise at least part of a cell-binding motif.

The invention also relates to a method of detection of a cytolysin, which has particular application in the diagnosis of pneumococcal infections.

### Background of the invention

A major cause of morbidity and mortality in humans is a group of pathogens which include the pneumococcus (*Streptococcus pneumoniae*) which causes various invasive diseases such as sinusitis and otitis media as well as the life-threatening diseases pneumonia, meningitis and bacteremia. The current pneumococcal therapies include antibiotics such as penicillin. However, resistance of pneumococcus strains to penicillin and other antimicrobial agents is prevalent. Studies have suggested that in developing countries five million children under the age of five die each year from pneumonia, the single most causative agent being the pneumococcus.

Although the pneumococcus capsule is an important virulence factor, several proteins are elaborated by the pneumococcus which are important in the pathogenesis of pneumococcal disease, one such protein being pneumolysin. Pneumolysin belongs to the thiol-activated family of cytolysins, produced by four genera of Gram positive bacteria. Pneumolysin's role in the virulence of the pneumococcus has been shown in both murine and guinea pig models of pneumococcal infection (Paton, J.C., Andrew, P.W., Boulnois, G.J., and Mitchell, T.J. 1993. "Molecular analysis of the pathogenicity of Streptococcus pneumoniae: The role of pneumococcal proteins". Annu. Rev. Microbiol.47: 89-115).

Molecular studies of pneumolysin and other cytolysins (op. cit.) have revealed various sequences including a cysteine motif common to all thiol-activated toxins so far sequenced, and various critical residues within this cysteine motif such as Cys 428, Trp 433, Glu 434 and Trp 435 in pneumolysin, as well as other residues in pneumolysin such as His 367 and Asp 385, which substitution can have a dramatic effect upon the activity of the toxin. Although various functional domains in pneumolysin have been described, the cell-binding site, which is potentially of the greatest therapeutic and diagnostic benefit, has not been identified.

The diagnosis of the pneumococcal infection mainly relies on the isolation of the pneumococcus from biological specimens, but this has two important limitations:
- the auto-lysis of the bacterium (spontaneous or induced by antibiotics), and
- the unavoidable slowness involved on its growth and identification *in vitro*: ≧48 hours.

Furthermore, serological diagnosis is necessarily slow since a time interval long enough is required for producing an immune response, and pairs of time-spaced serum samples are also required.

With the aim of obviating these drawbacks, more sensitive, specific and faster alternative diagnostic methods have been searched. The methods which potentially and advantageously meet today these conditions are those based on the rapid detection of bacterium components in both body fluids and infection sources where the pneumococcus is pathogenically present. Among these methods are immunoassays of different types: counter-immunoelectrophoresis, agluttination and/or co-agluttination, and enzyme linked immunosorbent assay (ELISA). The last is substantially the most sensitive (Holloway, Y., Snijder, J.A.M., and Boersma, W.G. 1993. "Demonstration of circulating pneumococcal immunoglobulin G immune complexes in patients with community-acquired pneumonia by means of an enzyme linked immunosorbent assay". J. Clin. Microbiol. 31: 3247-3254).

Hence therapies and diagnostic tests effective for pneumolysin and other cytolysins are extremely desirable as they may significantly reduce the impact of pneumococcal disease. Such therapies or diagnostic tests could comprise antibodies, e.g. monoclonal or polyclonal, or immunogenic polypeptides could be used individually or in combination with one-another or with other agents.

EP-A-0331034 describes diagnostic or therapeutic agents attached to two or more different antibody species which are reactive with different epitopes on the target cells but which have non-overlapping patterns of cross-reactivity. These agents may be attached to monoclonal antibodies which bind to cancer cells.

EP-A-0351789 describes cell lines which produce monoclonal antibodies capable of binding to human cachectin epitopes with enhanced neutralization capability *in vivo*.

WO-A-90/06951 describes a pneumococcal vaccine obtained from an altered pneumolysin by reason of one or more amino acid substitutions, and which also can be obtained from a recombinant clone including a replicon and a DNA sequence encoding the altered pneumolysin.

### Summary of the invention

A reliable and highly specific antigen diagnosis of the pneumococcus should ideally be based on a component which:
- Is present in all the isolated strains;
- Does not exhibit antigenic diversity and/or variability, and
- Has exclusive epitopes, not shared (absence of cross-reactivity) with antigenic components of other bacteria.

The immunoassays of the state of the art concern the identification of capsular polysaccharides or the C-polysaccharide of the cell wall, but none of these meets the required criteria.

By contrast, pneumolysin:
- Has been found in all the studied isolations of the pneumococcus;
- The gene encoding pneumolysin exhibits very reduced sequence variability; and
- Has own epitopes, not shared with other members of the family of bacterial toxins with which it keeps a high gene and amino acid sequence homology, such as streptolysin O.

Streptolysin O (SLO), produced by *Streptococcus pyogenes*, has some immunological resemblance with pneumolysin (PLY). It is also known that PLY shares with human C reactive protein (huCRP) two short regions in its sequence of amino acids.

The generation of monoclonal antibodies (MAbs) specific to PLY has a triple interest:
- From the point of view of basic research MAbs are deemed highly important biological tools to deep in the molecular characterization of the toxin and its structure-function relationships.
- Specific MAbs for exclusive epitopes of PLY could be used as diagnostic reagents of pneumococcal infections, in ELISA-type or immunoblotting assays, applied to biological samples from patients. Exclusive specificity of the said MAbs could mainly avoid risk of misdiagnosis from potential confusion with SLO.
- Pneumolysin and some pneumolysoids have been shown to confer protection-inducing capability against experimental pneumococcal infections. Therefore, specific MAbs for certain functional domains of the toxin could be equally protective and have, consequently, potential therapeutic applications in their original, derivative or modified forms thereof.

An object of the invention is the generation of hybridomas and the production and characterization of the corresponding monoclonal antibodies specific to pneumolysin, which could be used with several purposes, including diagnostic, prophylactic and therapeutic uses.

Also the invention identifies amino acid sequences required for cell-binding and cytolysis.

Another object of the invention is the detection of a cytolysin (pneumolysin) particularly in biological samples from patients, with the aim of pneumococcal infection diagnosis, by means of enzyme immunoassays with specific monoclonal antibodies. These antibodies do not recognize SLO or huCRP under assay conditions. The diagnosis method of the invention shows absolute specificity, and it is rapid and very sensitive.

According to the invention there is provided a polypeptide for use as the whole or part of a cell-binding motif, isolated and purified from nature and/or synthesised, comprising the amino acid sequence DKXE, where X is any amino acid.

Also the cell-binding motif may be of, at least, any of the following amino acids, DKXEXX, XDKXEXX, DKIE, DKIESF, GDKIESF or EDKVEND.

Also, the cell-binding motif may be a cytolysin cell-binding motif. The cytolysin may be another thiol-activated toxin such as perfringolysin O.

Any one of the preceding polypeptides or a derivative thereof may comprise part of a therapeutic or diagnostic composition in combination with a pharmaceutically acceptable carrier, diluent or excipient.

The derivative may comprise an antibody or a fragment or a derivative thereof specific to the polypeptide; the antibody may be a monoclonal or polyclonal antibody.

The composition may be used in conjunction with another agent or agents; the agent may comprise an antibody or capsular polysaccharide.

Hence therapeutic or diagnostic compositions may be generated, based upon the isolated and purified amino acid sequences of the present invention, and these compositions may be used to neutralise the cytolysins produced by e.g. PLY and perfringolysin O (PFO), thus reducing the threat to life provided by e.g. pneumonia. These compositions may comprise monoclonal or polyclonal antibodies as previously described, or they may comprise e.g. an immunogenic peptide according to the present invention in conjunction with an antibiotic. Hence protection may be generated against both the cytolysin produced by the pathogen as well as against the pathogen itself, thereby overcoming the problems of the present therapies.

Since the binding of cytolysins is an important step in the lysis of cells by this class of toxin, the cell-binding region of PLY was previously sought. In order to do this a panel of MAbs was generated and their ability to neutralise the cell-binding and haemolytic activity of PLY assayed. In order to further characterise the MAbs, a series of truncates of PLY was generated and the reaction of the antibodies with the truncates assayed, together with the cell-binding ability of the truncates.

### Description of specific embodiments, figures and tables of the invention

### Antigens:

Recombinant PLY was expressed in *Escherichia coli* and purified from cell extracts using standard techniques. Protein homogeneity was confirmed by sodium dodecyl sulphate polyacrylamide gel electrophoresis (SDS-PAGE).

### Antibody production:

BALB/c mice were hyperimmunized with recombinant pneumolysin. After applying different criteria of immunization, hybridomas of splenocytes and mouse myeloma Sp2/0 cells were generated by standard techniques.

Along the cloning and expansion process of secretor hybridomas, supernatants of the corresponding cultures were assayed by indirect ELISA against recombinant PLY.

Selected hybridomas were subcloned twice by limiting dilution. From the date of the second subcloning the hybridomas have been shown to be stable regarding their initial characteristics, including the production of anti-pneumolysin MAbs (antiPLY).

MAbs were purified by thiophilic adsorption chromatography on an AFFI-T^{(R)} column following the manufacturer's instructions (Kem-En-Tec, Copenhagen, Denmark).

The subisotype of the MAbs generated by the subcloned hybridomas was determined by indirect ELISA, in enzyme immunoassay (EIA) plates prepared with PLY and using a commercial kit *ad hoc* of Boehringer Mannheim.

The anti-pneumolysin MAbs, hereinafter identified as antiPLY-1, antiPLY-2 and antiPLY-3 were partially characterised through diverse techniques. Their properties are shown in brief in Table 1.

Purified myeloma protein MOPC-21 (Sigma) or anti-KLH monoclonal (IgG1, kappa generated by the inventors), were used as negative controls.

Mouse monoclonal 2-5B was used as a positive control for antibody reactivity with SLO.

### ELISA and immunoblotting tests:

ELISA and immunoblotting assays were carried out by standard techniques. For ELISA assays the enzymatic system employed was horseradish peroxidase; optical absorbance was measured at 492 nm (620 nm of reference) and substrates used were OPD (Sigma) and hydrogen peroxide. For immunoblotting tests, alkaline phosphatase was used, and a mixture of BCIP and NBT (Boehringer Mannheim) as substrate. Results were read with the naked eye.

All tests were run in triplicate.

### Estimations of Mab relative affinity:

Relative affinity of the MAbs was assessed in triple analysis by ELISA assays in which a determined amount of MAbs competed between 1 µg/well of plate adsorbed PLY and serial 1:10 dilutions of free PLY in solution.

### Inhibition of haemolytic activity:

Toxin neutralization assays were done in standard U-bottomed microtitre plates. Haemolytic activity of PLY batches was assessed immediately before the neutralization test. The end point of this assay was taken as the well in which 50% of the red cells were lysed. A stock toxin dilution in phosphate buffer 10 mM with 1% of bovine serum albumin and 0.1% of sodium azide (PBS-BSA-AS), pH 7.3, corresponding to twice said end point, was prepared. For the neutralization tests, 25 µl of serial two-fold dilutions of MAbs were added across 12 wells. The first well contained 1 µg of MAb. 25 µl of stock toxin (equivalent to 1 haemolytic unit) were added to each well and incubated at 37° C for 30 min. 50 µl of 1.6% sheep red blood cells in PBS were added and incubated at 37° C for 30 min. The titre of the antibody was taken as the highest dilution of MAb which completely inhibited haemolysis. All tests were done in triplicate.

### Cross-inhibition ELISA tests:

MAbs were allocated to various complementation groups on the basis of cross-inhibition ELISA studies.

MAbs were biotinylated according to standard procedures and titred against PLY.

EIA plates were coated with PLY (1 µg/well), and blocked with BSA. 100 µl of 1/10 serial dilutions of inhibitor MAb, starting at 10 µg/well, were added and incubated at 37° C for 2 h. Then 100 µl of biotinylated MAb were incubated for another 2 h period at 37° C. The binding to PLY of the labelled MAb was later estimated by incubation with horseradish peroxidase conjugated to streptavidin (Pierce) at a dilution of 1/10,000 for 2 h at 37° C.

All MAbs were challenged against each other both ways.

Absorbances which were obtained corresponded to those measured after subtraction of blank mean optical density. For any given MAb, the inhibition positive-control was taken as the biotinylated antibody challenged by 10 µg of the same unlabelled preparation. Wells into which no inhibitory antibody was added were considered as inhibition-negative controls; thus their mean optical density corresponds to 100% binding of the labelled MAb. The percentage inhibition afforded by any interfering MAb was calculated with reference to that absorbance.

MAbs were considered to bind to the same region of PLY, and therefore to be members of the same complementation group, if the inhibition was more than 50%.

### Inhibition of binding of PLY to red blood cells:

To assay cell-binding, dilutions of the toxin, from 260 to 3 ng/ml, were made in 1.5 ml of Hanks Buffered Saline (HBS) (GibcoBRL). 1 µg of MAb was added and the tubes incubated at 37° C for 1 h and then cooled to 4° C when 1.5 ml of 2% sheep RBC at 4° C were added. After a further incubation at 4° C for 30 min the cells were washed 3 times in ice cold HBS, then lysed in water. The membranes were harvested and washed twice in water by centrifugation at 13,000 rpm in a microfuge and then resuspended in sodium dodecyl sulphate-polyacrylamide gel electrophoresis (SDS-PAGE) loading buffer. SDS-PAGE was done, after which proteins were transferred to nitrocellulose membranes (Sartorius). The membranes were incubated in 5% (w/v) skimmed milk powder in PBS for 60 minutes, washed in PBS and incubated with 1/1000 dilution of rabbit anti-pneumolysin antiserum in PBS for 40 minutes. The membranes were washed, incubated for 60 minutes in 1/2000 goat anti-rabbit antiserum (Sigma) and then extensively washed in PBS. The proteins recognized by the anti-pneumolysin antiserum were visualised using Enhanced ChemiLuminescence reagents (Amersham) following the manufacturer's instructions.

A series of truncated versions of PLY were made, in which varying numbers of amino acids were deleted from the C-terminal, and the cell-binding and haemolytic activities of the truncates were assayed.

The polymerase chain reaction was used to generate the truncates using a cloned PLY gene template and primer which would produce the required truncate. Amplification products were cloned into vector pGEX2T (Pharmacia) before transformation into *Escherichia coli* strain JM101. Use of pGEX2T produced fusion proteins which consisted of the PLY truncate joined to the C-terminal of glutathione-S-transferase (GST) of *Schistosoma japonicum*. The proteins were separated by proteolytic cleavage with thrombin and purification of fusion protein from a crude cell-lysate was achieved by affinity chromatography on glutathione-sepharose beads. Recombinant PLY expressed as a free protein was also produced.

### Cell-binding assay:

Dilutions of the truncates, from 260 to 3 ng/ml, were made in 3 ml of Hanks buffered salts solution (HBSS) containing 0.2% (v/v) sheep erythrocytes and incubated in an ice-water bath for 30 minutes. A negative control of the truncate without erythrocytes was included in each experiment. The cells were washed three times in ice-cold HBSS, then lysed in water. The membranes were harvested and washed twice in water by centrifugation at 13,000 rpm in a microfuge and then resuspended in SDS-PAGE loading buffer. SDS-PAGE was done, after which proteins were transferred to nitrocellulose membranes. The membranes were incubated in 5% (w/v) skimmed milk powder in PBS for 60 minutes, washed in PBS and incubated with rabbit anti-PLY antiserum, diluted 1/1000 in PBS, for 40 minutes. The membranes were washed, incubated for 60 minutes in 1/2000 goat anti-rabbit antiserum and then extensively washed in PBS. The proteins recognized by the anti-pneumolysin antiserum were visualised using Enhanced ChemiLuminescence reagents (Amersham) following the manufacturer's instructions. The results were quantified using an LKB Ultrascan XL densitometer.

### Haemolyitic assay:

The haemolytic activity of the proteins was assayed as follows. Serial, two-fold dilutions of 50 µl of the solution to be analysed were made in PBS in a microtitre plate. 50 µl of 1.5% (v/v) sheep erythrocytes in PBS was added to each well. The plate was incubated at 37° C for 30 minutes. The haemolytic activity was defined as the inverse of the dilution of 50 µl of the sample protein that caused 50% haemolysis. To determine the inhibitory effect of anti-PLY antiserum, 50 µl of toxin were incubated with 50 µl of neat antiserum at 37° C for 30 minutes, before being assayed for haemolytic activity.

### Reaction of MAbs with truncated versions of PLY:

To locate regions of the PLY molecule recognised by selected MAbs such antibodies were tested for their ability to recognise the PLY truncates. The extracts were subjected to SDS-PAGE followed by western blotting as described above for the binding experiments. MAbs were then tested for their ability to recognise the truncates using the full length PLY molecule expressed in the form of GST-fusion as a positive control.

### Results:

As shown in Table 1, the three antibodies react with PLY in ELISA and in immunoblotting, what suggests that all three recognize continuous epitopes in the toxin molecule. Under similar experimental conditions, the three show a comparable degree of reactivity in immunoblotting, what it is not true in ELISA assays.

None of them reacts with SLO in ELISA. AntiPLY-3 weakly recognises this toxin in immunoblotting. However, when SLO is submitted to more drastic denaturating conditions (heating to 100° C for 30 minutes), the reaction is 3+, what indicates that this MAb recognises an internal epitope, no easily accessible, in the SLO molecule.

Furthermore, the three MAbs block the haemolytic activity of PLY, also exhibiting different capability.

**TABLE 1**

| Characteristics of the anti-pneumolysin monoclonal antibodies (a) | | | | | |
|---|---|---|---|---|---|
| | Reactivity with | | | | Neutralizing capability (ng of monoclonal) |
| | PLY | | SLO | | |
| Monoclonal (a) | ELISA | IB | ELISA | IB | HU of PLY (b) |
| antiPLY-1 | 8 | 3+ | - | - | 31.2 |
| antiPLY-2 | 2 | 3+ | - | - | ≦0.5 |
| antiPLY-3 | 5 | 3+ | - | ± | 1 |
| Control | - | - | - | - | - |
| a: All they are murine monoclonals IgG1, kappa. PLY: Pneumolysin. SLO: Streptolysin O. ELISA: 1 µg/well of antigen and 50 ng of monoclonal. Control was a monoclonal of the same subisotype without reactivity for PLY. IB: Immunoblotting developed with 500 ng of antigen and 5 µg of monoclonal. b: 1 HU (haemolytic unit) is defined as the amount of toxin required to lyse 50% of the tested erythrocytes. -: No colour development. Optical absorbances were measured after subtraction of blank mean optical densities. Absorbance values from 0.01 to 0.60 were quantified onto a 1-10 range level matrix. ±: Weak colour development. 3+: Strong colour development. -: 1 µg of monoclonal has no neutralizing capability. | | | | | |

Relative affinities of the MAbs antiPLY-1, antiPLY-2 and antiPLY-3 were 4.5x10⁻¹¹ M, 4.7x10⁻¹⁰ M and 8.5x10⁻¹⁰ M, respectively.

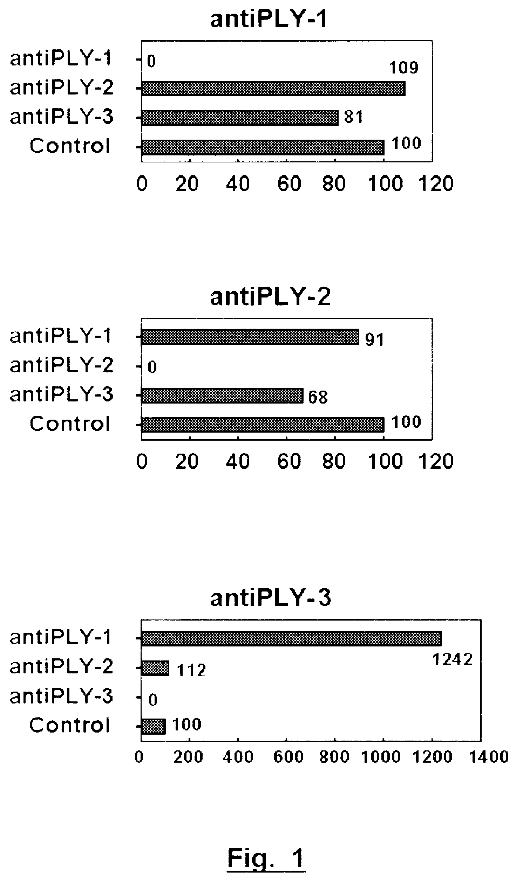

Figure 1 is a diagram showing the results of the cross-inhibition ELISA tests as reaction percentages obtained through the application of the previously described criteria.

On top of each diagram it is indicated the biotilynated MAb challenged by the MAbs mentioned at the left. As represented, each assayed MAb belongs to a specific complementation group since no inhibition was observed among them; the percentages observed were always higher than 50%. This means that said three MAbs recognize distinct epitopes in the PLY molecule. It should be also observed that antiPLY-1 has a strong positive modulation over the subsequent interaction of antiPLY-3 with PLY (note a far larger scale for diagram of antiPLY-3); a reciprocal effect was not observed.

A second evidence of the fact that said three MAbs recognize different epitopes in the PLY molecule was obtained from sandwich ELISA tests by capture of free PLY. Each one was used as first capture MAb and assayed with the biotilynated form, as a reporter, of any of the other two MAbs. Assays carried out in triplicate were always positive, as expected. Controls with a capture MAb of irrelevant specificity for PLY were negative.

Aliquots of the murine hybridomas producing the monoclonal antibodies antiPLY-1, antiPLY-2 and antiPLY-3 have been deposited on 27th January 1994 with the International Depositary Authority, Collection Nationale de Cultures de Microorganismes Institut Pasteur (CNCM), 28 rue du Docteur Roux, F-75724 Paris Cedex 15, receiving Accession Numbers I-1394, I-1395 and I-1396 respectively.

In summary, there have been obtained three murine hybridomas producing three different MAbs which
- have distinct affinity for PLY;
- recognize different epitopes in the PLY molecule;
- have proved useful in sandwich ELISA tests for the immunodetection of PLY in biological samples, and
- have neutralizing capability of the haemolytic activity *in vitro* of PLY.

Experiments with the truncates showed that the cell-binding of PLY I-465 (missing the six carboxy-terminal amino acids) was greatly reduced (Table 2), as was its haemolytic activity, indicating that the six carboxy-terminal amino acids form at least part of a cell-binding motif. The binding of the antibodies to the truncates (Table 3) showed that antiPLY-3 only reacts weakly with the truncate lacking the C-terminal six amino acids, suggesting that it recognises an epitope on the extreme C-terminal of PLY.

Pre-incubation of PLY with antiPLY-3 prior to adding the mixture to sheep erythrocytes (Table 3) neutralised the ability of PLY to bind the sheep erythrocytes.

Hence antiPLY-3 recognises an epitope on the extreme C-terminal of PLY and neutralises its haemolytic and cell-binding activities. Pre-incubation of PLY with antiPLY-3 inhibits the cell-binding activity of PLY. Therefore the epitope recognised by antiPLY-3 appears to comprise at least part of a cell-binding motif.

This is confirmed by a recombinant pneumolysin fusion protein with the six carboxy-terminal amino acids deleted having significantly reduced cell-binding and haemolytic activities.

The seven C-terminal amino acids of PLY are EDKVEND, five of which are charged. Further experiments with another cytolysin, perfringolysin O (PFO), shows that antiPLY-3 recognises a linear epitope on PFO, inhibiting PFO's cell-binding ability. PFO has a similar sequence (GDKIESF), the consensus sequence between the two being DKXE, it appearing that the cell-binding motif of these cytolysins comprises at least the sequence DKXE, and possibly DKXEXX or XDKXEXX. Thus the perfringolysin O sequence may be DKIE or DKIESF or GDKIESF.

Throughout this document peptides are identified using a single letter to represent each separate amino acid. Each letter is the conventional single letter symbol used for amino acids.

**Table 2**

| Cell-binding and haemolytic activity of recombinant PLY | | |
|---|---|---|
| Portion of molecule (recombinant fusion construct unless otherwise stated) | Binding Activity | Haemolytic Activity |
| I-471 (recombinant PLY) | 100 | 100 |
| I-471 | 100 | 100 |
| I-465 | 2 | 0.2 |
| I-450 | 0 | 0 |
| I-441 | 0 | 0 |

Numbers for portion of recombinant molecule represent the amino acid residues from native PLY present in the truncate.

Binding activity and haemolytic activity are represented as a percentage of the activity of the full length construct.

**Table 3**

| Reaction of monoclonal antibodies: ability to neutralise PLY's haemolytic activity, ability to bind to truncates of PLY and to PFO, and ability to inhibit cell-binding of PLY pre-incubated with monoclonal antibody. | | | | | | | | | | |
|---|---|---|---|---|---|---|---|---|---|---|
| Antibody | Neutralizing ability | Amino acid number | | | | | | | Perfringolysin O PFO | Cell binding |
| | | 1-263 | 1-419 | 1-430 | 1-441 | 1-450 | 1-465 | 1-471 | | |
| PLY-2 | + + + | - | + | + | + | + | + | + | + | + |
| PLY-3 | + + + | - | - | - | - | - | ± | + | + | - |
| PLY-1 | + | - | ± | + | + | + | + | + | - | - |
| Poly | + + + | + | + | + | + | + | + | + | + | |

The invention also relates to a method for detection of a cytolysin in biological samples by a sandwich ELISA test, comprising the following steps:
- preparation of enzyme immunoassay plates with first capture monoclonal antibody(ies),
- addition of the biological sample to be assayed,
- addition of second antibody(ies) as reporter(s), and
- development of a reaction quantifiable by colour-measuring.

Compared with the state of the art, the detection of pneumolysin applied to the diagnosis of pneumococcal diseases or cytolysin involved (cytolytin) infections, is:
- Very fast since it can be developed in less than 7 hours.
- Highly specific for the pneumococcus in such a way which excludes potential confusion with other microorganisms, and
- Highly sensitive since PLY is detected in quantities of picograms.

A practical embodiment of the method of the invention will be described.

Flat-bottomed 96-well polysterene EIA plates (Costar) are coated with 1 µg/100µl-well of solution of the first capture antibody in carbonate-bicarbonate buffer (0.05 M, pH 9.6). Plates are incubated at 37° C for 6 hours.

Then the wells are sucked and refilled with 200 µl of a bovine serum albumin solution (1% w/v) in phosphate buffer 10 mM, with 0.1% of sodium azide (solution PBS-BSA-AS, pH 7.3).

Plates are incubated at 37° C for 1 hour and then cooled, a 4° C, for at least 12 hours.

Once prepared, plates are washed 3 times with phosphate buffer added with 0.01% Tween 20 (PBS-T). Then 100 µl/well of biological samples are added and incubated at 37° C for 2 hours.

As positive antigen control a first series of wells is incubated with different concentrations of recombinant PLY; as negative control a second series is incubated with SLO. A third series without any antigen and refilled with PBS-BSA-AS, is used as blank for final measurement of optical density.

Plates are washed again three times.

Then 100 µl/well of the second biotilynated MAb in PBS-BSA-AS are added. Plates are incubated for 2 h at 37° C.

After washing the plates, wells are then refilled with 100 µl of ImmunoPure^{(R)} horseradish peroxidase conjugated streptavidin (Pierce), dilution 1:10,000 in PBS-BSA-T. Incubation at 37° C for two hours.

After corresponding washings, wells are incubated with 100 µl of solution substrate OPD (Sigma) and hydrogen peroxide in citrate-phosphate buffer, pH 5.0, at 37° C for 30 minutes in the dark.

Finally, optical absorbance per well is read at 492 nm (620 nm of reference) on a ELISA plate reader.

A test is considered as positive when having an optical absorbance at least double than the corresponding to wells without any antigen (blanks).

Carrying out accurately the above mentioned method the following recombinant PLY concentrations were tested: 200, 100, 50, 25, 12.5 and 6.25 ng/ml. SLO (Sigma, S 5265) and huCRP (ICN Biochemicals, Cat. No. 152315) were tested at 10 µg/ml.

No reactivity was observed with 1 µg of SLO or huCRP. By contrast, quantities of ≦625 picograms (minimum amount tested) of pneumolysin were clearly detectable.

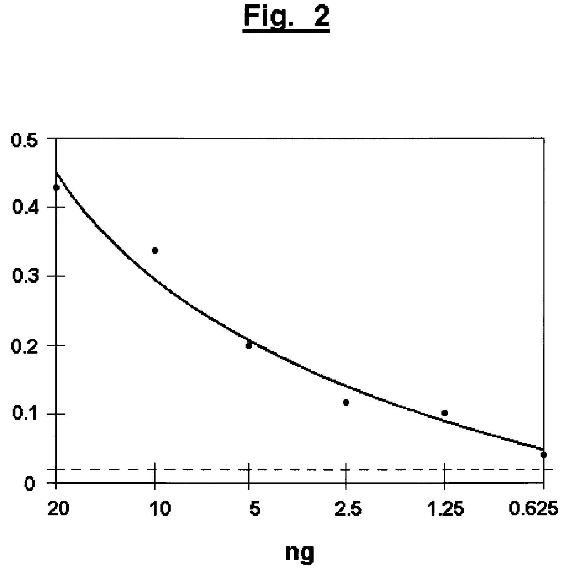

Figure 2 is a diagram showing absorbance variation at 492 nm according to the quantity of pneumolysin tested per well.

Above data are referred to purified preparations of the implicated antigens. Tests were also negative when supernatants of cultures in stationary phase (10¹² CFU ml⁻¹) of *S. pyogenes* were analysed; in contrast, analogue culture derivatives of *S. pneumoniae* (10⁸ CFU ml⁻¹) were positive.

The results obtained show:
- That the proposed test can be used for the capture and detection of native pneumolysin in biological samples of patients suffering pneumococcal infections.
- That it is specific, since false positives mainly arising from SLO and huCRP detection are excluded.
- That detection of pneumolysin in a biological sample would be an unchallenged sign of the, present or previous, presence of the pneumococcus, even if isolation of the pneumococcus were negative for any reason.
- That the test is highly sensitive, since it allows the detection of pneumolysin in picogram level quantities.

## Claims

1. A monoclonal antibody comprising the antiPLY-1 monoclonal antibody.

2. The cell line designated CNCM Accession Number I-1394 which produces the antiPLY-1 monoclonal antibody according to claim 1.

3. A monoclonal antibody comprising the antiPLY-2 monoclonal antibody.

4. The cell line designated CNCM Accession Number I-1395 which produces the antiPLY-2 monoclonal antibody according to claim 3.

5. A monoclonal antibody comprising the antiPLY-3 monoclonal antibody.

6. The cell line designated CNCM Accession Number I-1396 which produces the antiPLY-3 monoclonal antibody according to claim 5.

7. A cell line generated totally or partially from any one of the cell lines of claims 2, 4 and 6.

8. A therapeutic or diagnostic composition comprising any part or derivative of the cell lines of claims 2, 4 and 6.

9. A therapeutic or diagnostic composition comprising an antibody, fragment or derivative thereof capable of blocking the epitope recognised by the monoclonal antibodies of claims 1, 3 and 5.

10. A therapeutic or diagnostic composition comprising an antibody, fragment or derivative thereof of any one of claims 1, 3 and 5 in combination with a pharmaceutically acceptable carrier, diluent or excipient.

11. A composition according to claims 8, 9 and 10 capable of inhibiting the cytolytic properties of a cytolysin.

12. A composition according to claim 11 wherein the cytolysin is pneumolysin.

13. A composition according to claim 11 wherein the cytolysin may be another thiol-activated toxin such as perfringolysin O.

14. The amino acid sequence of at least DKXE wherein X is any amino acid, which comprises at least part of a cytolysin binding motif.

15. The amino acid sequence DKIE according to the preceding claim.

16. The amino acid sequence according to claims 14 or 15 wherein it comprises at least part of a binding motif of perfringolysin O.

17. The amino acid sequence EDKVEND according to claim 14.

18. The amino acid sequence according to claim 17 wherein it comprises at least part of a binding motif of pneumolysin.

19. A therapeutic or diagnostic composition comprising the polypeptide of any one of the preceding claims in combination with a pharmaceutically acceptable carrier, diluent or excipient.

20. An antibody or a fragment or a derivative thereof specific to an epitope of the amino acid sequence of any one of claims 14 to 18.

21. An antibody according to claim 20 wherein it is a monoclonal, polyclonal or recombinant antibody.

22. A method for detection of a cytolysin in biological samples comprising at least the following steps:
(a) preparation of enzyme immunoassay plates with capture antibody(ies) specific to the cytolysin,
(b) addition of the biological sample to be assayed,
(c) addition of reporter antibody(ies) specific to the citolysin, and
(d) development of a reaction quantifiable by measuring an optical density.

23. A method for detection of a cytolysin, based on the use of monoclonal antibodies specific for the cytolysin in a sandwich ELISA test, comprising at least the following steps:
(a) preparation of enzyme immunoassay plates with monoclonal antibody(ies) specific to the cytolysin, acting as capture antibody(ies), and
(b) addition of second monoclonal antibody(ies) distinct from the capture monoclonal antibody(ies), specific to the cytolysin and used as reporter antibody(ies).

24. A method for diagnosis of infections after detection of the cytolysin in biological samples, according to claim 22.

25. A method for diagnosis of infections after detection of the cytolysin in biological samples based on the use of monoclonal antibodies specific to the cytolysin in a sandwich ELISA test, according to claim 23, comprising at least the following steps:
(a) preparation of enzyme immunoassay plates with capture monoclonal antibody(ies) specific to the cytolysin,
(b) addition of the biological sample to be assayed,
(c) use of second antibody(ies) specific to the cytolysin and distinct from the first one(s), as reporter antibody(ies), and
(d) development of a colour reaction quantifiable through the measurement of the optical density.

26. A method according to either one of claims 22 to 24 wherein the cytolysin is pneumolysin.

27. A method according to either one of claims 22 to 24 wherein the cytolysin is perfringolysin O.

28. A method according to either one of claims 24 and 25 wherein the organism is a pneumococcus and the cytolysin is pneumolysin.

29. A method according to either one of claims 24 and 25 wherein the organism is *Clostridium perfringens* and the cytolysin is perfringolysin O.
